# EUROPEAN PATENT APPLICATION

(11) **EP 3 804 766 A1**
(43) Date of publication of application: **14.04.2021**
(21) Application number: 19819859.0
(22) Date of filing: 11.06.2019
(51) Int. Cl.: A61K 48/00, A61K 47/10, A61K 47/34, A61P 25/28

(54) **THERAPEUTIC AGENT FOR ALZHEIMER'S DISEASE**

(30) Priority: 11.06.2018 JP 2018111488
(71) Applicant: Mitsuyama, Fuyuki, Aichi-gun Aichi, 470-0166 (JP)
(72) Inventor: Mitsuyama, Fuyuki, Aichi-gun Aichi, 470-0166 (JP)
(74) Representative: Schlich, George
(86) International application number: PCT/JP2019/023176
(87) International publication number: WO 2019/240145

(57) **Abstract**

[Problem] To provide: an actually effective therapeutic method which is for dementia of Alzheimer's disease, and which is a causal treatment rather than a symptomatic treatment; and a therapeutic substance for dementia of Alzheimer's disease. [Solution] This substance alleviates symptoms such as dementia of Alzheimer's disease by overexpressing a microtubule-associated protein 1B gene in intracerebral neurons. In addition, as methods to solve the problem above, there are mainly two methods: a method for intracerebral administration of a gene therapy vector bound to a microtubule-associated protein 1B gene; and a method for binding a microtubule-associated protein 1B gene to a blood-brain barrier (BBB) permeable nanomachine and administering intravascularly.

## Description

### Technical Field

The present invention relates to a therapeutic agent for dementia in Alzheimer's disease. More specifically, it relates to gene therapy which introduce and express a gene into nerve cells in the brain.

### Background Art

Although at present, it is known that the cause of Alzheimer's disease is the accumulation of amyloid in the brain, there are various theories and it is undecided how the nerve cells in the brain are damaged by the amyloid. Therefore, the causal therapy is almost limited to those targeting amyloid such as amyloid antibody (Non-Patent Literature 1), but it has not been successful. Therefore, symptomatic treatment is mainly performed.

A drug called donepezil can increase a neurotransmitter called acetylcholine in the brain (Patent Document 1, Non-Patent Document 2). However, since the synaptic neurotransmitter that controls memory is glutamate, acetylcholine is only a symptomatic treatment and can only slightly suppress the progression of early to mid-stage symptoms of Alzheimer's disease, and its effect is limited.

That is, most of these conventional symptomatic treatments have very little effect on dementia and have little clinical effect. It is no exaggeration to say that there is no drug or method that can effectively treat dementia in Alzheimer's disease.

As a solution to this problem, there is a treatment method using an amyloid antibody, which is considered to be a causal treatment, but it has the disadvantages of being less effective and having strong side effects such as cerebral edema, and it has been required to solve the problems.

### Related Art Documents

### Patent Literatures

Patent Literature 1: Japanese Patent Publication No. 0005562337

### Non-Patent Literatures

Non-Patent Literature 1: Biosci Biotechnol Biochem.; 78 (8): 1293-305 (2014)
Non-Patent Literature 2: J Alzheimers Dis. 2018 Apr 28. doi: 10.3233 / JAD-180159

### Summary of the Invention

### Problems to be solved by the invention

The problem to be solved is not to provide symptomatic treatment, but to provide a practically effective treatment method for dementia of Alzheimer's disease and a therapeutic substance thereof, which can be a causal treatment.

### Means to solve problems

According to the present invention, the following inventions are provided.

(1) Atherapeutic agent for dementia comprising any one of
i) DNA encoding microtubule-associated protein 1B (MBP1B);
ii) A gene encoding a protein having 70% or more homology with the DNA encoding MBP1B and having the equivalent function;
iii) DNA that hybridizes with the DNA encoding MBP1B under stringent conditions and encodes a protein having the equivalent function as the MBP1B protein.;
iv) DNA encoding a protein that has 60% or more identity with the amino acid sequence of the MBP1B protein and has the equivalent function as the MBP1B protein.

In the present invention, it provides methods and substances for improving symptoms such as dementia in Alzheimer's disease by overexpressing the microtubule-associated protein 1B gene or a gene homologous to it having the same or similar function in nerve cells in the brain. Further, the encoding gene means an open reading frame (ORF) or a coding region (preferably the total length), and means a DNA that expresses a functioning protein when it is introduced into a cell with a promoter which is functionally linked to the encoding gene. The promoter may include an internal ribosomal entry site (IRES), an enhancer, an insulator, or the like, if necessary as well. In the present invention, as a general rule, the full length of the ORF (of cDNA) is introduced, but DNA substitution, addition, deletion, etc. may be included as long as it has the equivalent function as a protein. Substitution, addition, or deletion, etc. of DNA is not limited as long as they have equivalent functions, but are 30% or less, more preferably 20% or less, still more preferably 10% or less of the full sequence, particularly preferably one to several nucleotide.

(2) The dementia therapeutic agent according to (1), which is functionally linked to a vector.

As a method for achieving (1), there are roughly two methods: a method of directly injecting a gene therapy vector bound to a microtubule-associated protein 1B gene into the brain; intravascular administration by binding the microtubule-associated protein 1B gene to a BBB (blood-brain barrier) cross-type nanomachine. Here, the one linked to the vector is provided.

(3) The therapeutic agent for dementia according to (1), which is characterized by being bound to a blood-brain barrier (BBB) blood-brain barrier nanomachine.

A method and substance for intravascular administration by binding the microtubule-associated protein 1B gene to a blood-brain barrier (BBB) cross-type nanomachine. The blood-brain barrier controls the transport of circulating blood and cranial nerve parenchyma, mainly by cerebral vascular endothelial cells. It takes in nutrients essential for brain activity, but significantly limits the delivery of drugs to the brain. Kazunori Kataoka and colleagues have succeeded in developing a blood-brain barrier crossing nanomachine that crosses the blood-brain barrier with significantly higher efficiency than existing technologies in response to the simple stimulus of changes in blood glucose levels and furthermore, that accumulates in nerve cells in the brain. If the microtubule-associated protein 1B gene is bound to this blood-brain barrier-crossing nanomachine and administered intravascularly, it is possible to express the microtubule-associated protein 1B gene in nerve cells in the brain simply by administering it intravascularly without the need for brain surgery.

(4) A method for treating dementia by expressing microtubule-associated protein 1B in brain cells.
(5) Use of the microtubule-associated protein 1B gene for producing a therapeutic agent for dementia.
(6) Microtubule-associated protein 1B gene used for the treatment of dementia.
(7) A method for treating dementia by administering the dementia therapeutic agent according to any one of (1) to (3).

(8) A method for treating dementia by introducing and expressing any of the following DNAs:
i) DNA encoding microtubule-associated protein 1B (MBP1B);
ii) A gene encoding a protein having 70% or more homology with the DNA encoding MBP1B and having the equivalent function
iii) DNA that hybridizes with the DNA encoding MBP1B under stringent conditions and encodes a protein having the equivalent function as the MBP1B protein;
   or
iv) A DNA encoding a protein having 60% or more identity with the amino acid sequence of the MBP1B protein and having the equivalent function as the MBP1B protein.

The site of introduction is preferably in the brain, but if the DNA can cross the blood-brain barrier, it may be injected into the arm or the like, introduced into the blood, and moved to the brain for expression.

(9) Use for manufacturing a drug for treating dementia of any one of
i) DNA encoding microtubule-associated protein 1B (MBP1B);
ii) A gene encoding a protein having 70% or more homology with the DNA encoding MBP1B and having the equivalent function;
iii) DNA that hybridizes with the DNA encoding MBP1B under stringent conditions and encodes a protein having the equivalent function as the MBP1B protein;
   or
iv) DNA encoding a protein having 60% or more identity with the amino acid sequence of the MBP1B protein and having the equivalent function as the MBP1B protein..

(10) Any one of DNA to treat dementia, consisting of
i) DNA encoding microtubule-associated protein 1B (MBP1B)
ii) A gene encoding a protein having 70% or more homology with the DNA encoding MBP1B and having the equivalent function.
iii) DNA that hybridizes with the DNA encoding MBP1B under stringent conditions and encodes a protein having the equivalent function as the MBP1B protein.
   or
iv) DNA encoding a protein having 60% or more identity with the amino acid sequence of the MBP1B protein and having the equivalent function as the MBP1B protein.

(11) The DNAaccording to (10), wherein the DNA is further linked to a vector. (12) The DNA according to (10), characterized by being bound to a blood-brain barrier (BBB) crossing nanomachine further.

### Effects of the Invention

The present invention has the effect of improving dementia in Alzheimer's disease.

### Brief Description of the Drawings

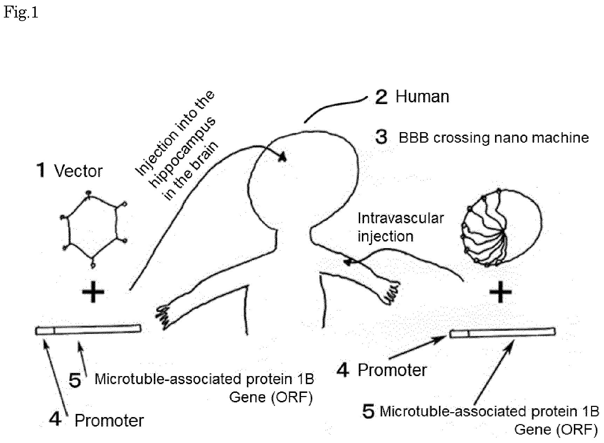

[Figure 1] Fig. 1 is a schematic view showing an overall picture of the present invention. (Example 1).
[Figure 2] Fig. 2 is a conceptual diagram of a method for producing a gene transfer substance of the present invention. (Example 1).
[Figure 3] Fig. 3 is a graph showing the effect of introducing the microtubule-associated protein 1B gene
[Figure 4] Fig. 4 is a photograph showing that dendritic spines expand when microtubule-associated protein 1B is expressed in cultured cells.
[Figure 5] Fig. 5 is a photograph and a diagram of microtubule-stained spines grown by introducing and expressing microtubule-associated protein 1B in cultured cells.
[Figure 6] Fig. 6 is a photograph of western-blotting and a graph showing the degree of it using an antibody of a protein representing the spines of the right hippocampus and the left hippocampus, by taking out the hippocampus of the model mouse brain one week after the injection of which right hippocampus of the model mouse was injected when the microtubule-associated protein 1B gene was injected into the brain (Example 2).

### DESCRIPTION OF THE EMBODIMENTS

In the present specification, dementia is a type of cognitive impairment, and refers to a state in which intelligence that has once developed normally is irreversibly reduced due to an acquired organic disorder of the brain. Dementia also develops in non-humans such as dogs and cats. In a narrow sense, it refers to "a state of acquired decline in intelligence ", but in this specification, it means syndromes accompanied by cognitive impairment including "memory" and "orientation" and "personality change" in addition to "intelligence".

In the present specification, dementia is often observed as a symptom in diseases such as Alzheimer's dementia, frontotemporal dementia, Parkinson's disease, Levy's body dementia, Huntington's disease, Kreuzfeld-Jakob's disease, muscle atrophic lateral sclerosis with dementia, or prion disease, but is not limited to these diseases. Recent studies have often reported that other diseases with dementia other than Alzheimer's disease often have spinal and / or synaptic loss and amyloid deposits. In other words, there are many commonalities in diseases showing dementia, and the treatment methods and therapeutic substances for dementia of Alzheimer's disease, which are mainly described in this specification, may be effective for dementia other than dementia of Alzheimer's disease.

In the present specification, Alzheimer's disease is a disease that generally develops in old age and is pathologically characterized by the deposition of amyloid in the brain, and whose main symptom is memory impairment.

In the present invention, microtubules are fibrous organelles found in axons, dendrites, and spines in nerve cells, by which cell process is formed such as axons, dendrites, and spines, and along which substances are transferred.

Microtubule-binding protein (MBP1B, which has the same meaning as microtubule-associated protein) is a protein that exists attached to the surface of fibrous microtubules and promotes stabilization and elongation of microtubules. Four types of microtubule-associated proteins are known: microtubule-associated protein 1A / 1B, microtubule-associated protein 2, and tau. In the present specification, unless otherwise specified, the DNA encoding MBP1B means a DNA containing the entire length of an open reading frame (ORF), and may be the ORF itself, or may have a linker or the like at the upstream or the downstream of the ORF.

In the present invention, gene overexpression means that a large amount of a gene of interest is produced in vitro; a promoter that enhances the degree of expression in the target tissue, organ, etc. is linked to the region encoding the protein so as to function; an expression cassette is also created with a carrier that facilitates introduction into cells such as vectors; the expression cassette is inserted into the target cell (gene transfer); by borrowing the mechanism of protein synthesis of preexisting cells; the protein is finally overproduced in the cells. In the present invention, overexpression also includes introducing an expression cassette into blood, crossing the blood-brain barrier, and overexpressing in brain nerve cells.

As used herein, a vector is a carrier that binds to a gene and puts the gene into the cell for the purpose of expressing the gene in the cell.

In the present specification, a plasmid refers to a circular double-stranded DNA that replicates independently of chromosomal DNA in bacteria such as Escherichia coli. Aplasmid into which a target gene is incorporated by utilizing this property is used to cause gene expression in various cells (also referred to as a plasmid vector).

In the present invention, the DNA used for the treatment of dementia (which is a translation of ninchisyo, but was unified by the Ministry of Health, Labor and Welfare as dementia because it is a word that insults patients) includes: i) DNA encoding microtubule-associated protein 1B (MBP1B); ii) DNA encoding a protein having 70% or more homology with DNA encoding MBP1B and having the equivalent function as MBP1B protein; iii) DNAthat hybridizes with the DNA encoding MBP1B under stringent conditions and encodes a protein having the equivalent function as the MBP1B protein; iv) DNA encoding a protein having 60% or more identity with the amino acid sequence of the MBP1B protein and having the equivalent function as the MBP1B protein. Dementia can be treated by introducing any of these DNAs into brain nerve cells and by expressing the MBP1B protein or a protein having an equivalent function in the brain nerve cells. The main object of the present invention is to provide a therapeutic agent for dementia, but it is considered that it may be effective in preventing dementia from the principle of the present invention.

Here, the DNA to be introduced and expressed preferably has 70% or more homology with the DNA encoding MBP1B and has the equivalent function as the MBP1B protein, but more preferably 75% or more and 80% or more., 85% or more, 90% or more, 91% or more, 92% or more, 93% or more, 94% or more, 95% or more, 96% or more, 97% or more, 98% or more, 99% or more homology may be used.

In addition, stringent conditions include, for example, conditions for washing at 68 °C, 0.1 × SSC, and 0.1% SSC after Southern hybridization.

Amino acid sequence identity of a protein which can be used in the present invention, having equivalent function with MBP1B protein, compared with amino acid sequence of the MBP1B protein is preferably 60% or more. More preferably, the identity may be 65% or more, 70% or more, 75% or more, 80% or more, 85% or more, 90% or more, 91% or more, 92% or more, 93% or more, 94% or more, 95% or more, 96% or more, 97% or more, 98% or more, 99% or more.

Whether or not the protein produced from the DNA homologous to the DNA encoding MAP1B has the equivalent function as MAP1B can be confirmed by introducing it into brain nerve cells and by conducting the Y maze test (Neuroscience. 2006 Dec 1; 143 (2): 395-405. Epub 2006 Sep 14. PMID: 16973297).

In the present specification, the blood-brain barrier means a tissue that can be said to be a biological barrier which has a function controlling the transport of substances between the circulating blood and the cranial nerve system, mainly by cerebral vascular endothelial cells, and selectively takes in nutrients essential for brain activity, while significantly limits the delivery of drugs to the brain.

In the present specification, a promoter refers to a sequence having a function of transcribing mRNA from a gene. Depending on the purpose, it may be DNA or RNA, but DNA is usually used. A promoter may have a cis element such as an enhancer or a silencer, but is not particularly distinguished in the present specification, and a promoter having an enhancer or the like is also referred to as a promoter. In addition, a promoter in which a cis element is bound at the upstream or the downstream of the core promoter can also be used.

The present invention is characterized by expressing microtubule-associated protein 1B in brain neurons (preferably in hippocampal neurons). Examples of promoters capable of expressing genes in brain nerve cells include, but are not limited to, CMV (cytomegalovirus) promoter, EF1α promoter, etc., and may be a combination of promoters and enhancers capable of expressing foreign genes in brain nerve cells.

Atypical embodiment of the present invention is shown.

The Map 1b gene containing a region encoding microtubule-associated protein 1B (MAP1B) can be cloned by a known method. For example, mRNA having a polyA chain is prepared from a tissue expressing the Map 1b gene (for example, brain tissue containing the hippocampus), and preferably, a poly T sequence with a linker (poly T is preferably about 18 to 30) is annealed to synthesize cDNAby reverse transcriptase. Using this cDNA, the DNA containing the region encoding the MAP1B protein can be amplified by PCR using a suitable primer, for example, using DNA having a sequence of about 20 to 30 bases in the region containing the start codon of the region encoding the MAP1B protein as the 5'-side primer, and using the poly T sequence with a linker is used as the 3'-side primer. The PCR conditions can be determined by a method well known to those skilled in the art depending on the length and properties of the primer. As the amplification enzyme (polymerase) to be used, it is preferable to use an enzyme having high fidelity (less error). Examples of highly accurate amplification enzymes include, but are not limited to, pfu, KOD polymerase, and the like. If necessary, the DNA of the PCR product is cloned into a plasmid to determine the base sequence, so that it can be confirmed whether or not there is mutation due to amplification.

Alternatively the Map1b gene can be cloned by screening a cDNA library or a genomic library by plaque hybridization or the like using an amplified DNA fragment of the full length or a part of the Map1b gene or a cloned Map1b gene DNA fragment as a probe. At this time, by lowering the stringency and by screening, a gene having homology with the Map1b gene can be found and cloned.

Linkers can be inserted by designing appropriate primers and amplify from start codon to termination codon or its downstream of Map1b gene which is obtained by PCR or by screening from a library. Preferably, a linker attached primer can be used during cDNA synthesis, a linker is attached after the next complementary strand synthesis, or a linker attached 5'-side primer can be also used during PCR to avoid extra work.

The DNA containing the full length of the region encoding the MAP1B protein obtained by these procedures can be ligated to the vector to function. For example, it is linked to a promoter of Lenti-X Vector (Lenti-X TM HTX Packaging System) of Clontech Inc. (for example, cytomegalovirus promoter, EF1α promoter, etc.) and the like, so as to function and introduced into cells or blood to produce the protein in the desired cell.

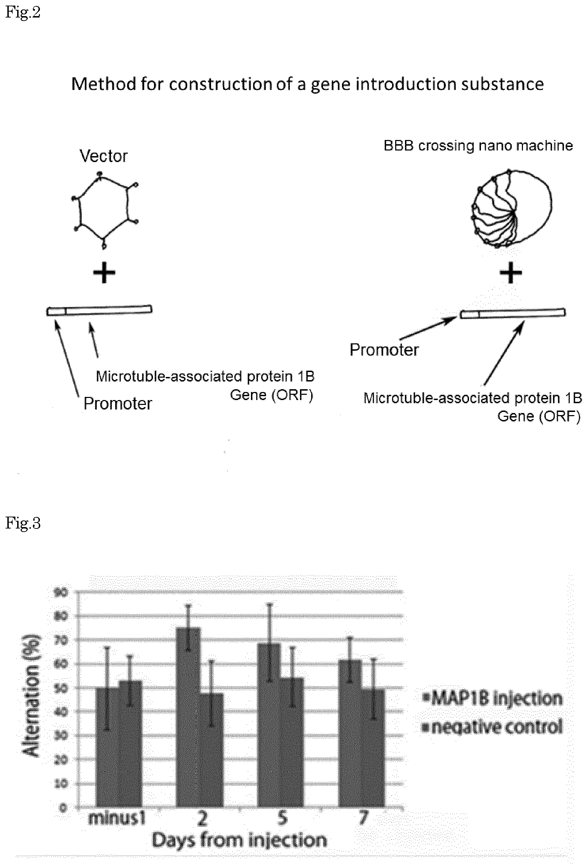

Fig3 shows a change in the degree of dementia in Alzheimer model mice measured by the Y maze test when a lentiviral vector bound to the microtubule-associated protein 1B gene was injected stereotactically into the right hippocampus by puncturing in the skull of an Alzheimer's disease model mouse. Binding of viral vectors improved the degree of expression of the microtubule-associated protein 1B gene in hippocampal neurons. Two days after the injection, the degree of dementia in the injection group was clearly improved (Fig. 3).

Alzheimer's disease is thought to occur when amyloid accumulates in the brain, which adversely affects nerve cells, but the mechanism has not been elucidated. It is thought that amyloid binds to microtubule-associated protein 1B protein, which inactivates microtubule-associated protein 1B protein, resulting in depolymerization (melting) of microtubules in the spines that form the posterior synapse and shortening of the spines to reduce the function of synapse. One week after local injection, the effect gradually diminished, probably because a model mouse with five gene mutations in the amyloid-producing gene was used to obtain faster results (Oakley, H.. et.al. J. Neurosci. 40, p10129-10140 (2009)). The more mutations there are, the sooner the symptoms appear. In human Alzheimer's disease, 20% have one gene mutation, and the rest have no gene mutation. It is believed that the very fast deposition of amyloid counteracts the effects of microtubule-associated protein 1B.

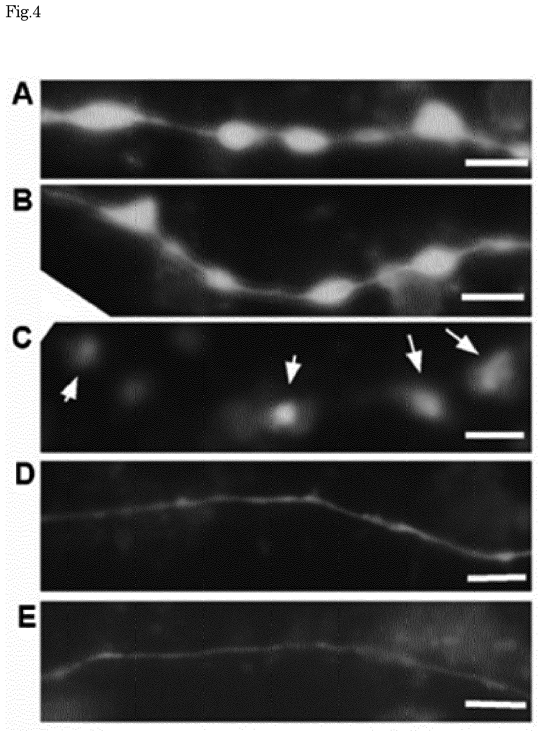

The microtubule-associated protein 1B gene bound to a lentiviral vector was introduced into primary cultured neurons derived from the hippocampus (where synapses were formed about 2 weeks after culturing) by the calcium phosphate method and was overexpressed. When the MAP1B gene bound to the lentiviral vector was added, the dendrite spines forming the posterior synapse expanded (AB in Fig. 4) about 10 times as much as those without the addition (DE in Fig. 4). D and E are normal dendrites and microtubule-associated protein 1B was not used. Only GFP (green fluorescein protein) was introduced, and the entire nerve cell was dyed green, where fine dendrites were dyed. Small bulges are normal spines, to which nerve cells from other sources input and form synapses. For A and B, in addition to GFP, microtubule-associated protein 1B bound with a lentiviral vector + GFP was introduced. The protrusions of the dendrites have become huge. In C, a gene in which the yellow pigment gene YFP95 was bound to the gene PSD95, which is a protein representing spines, was introduced. A spot of a gene indicating that it is a spine appears in the protrusion on the enlarged dendrite, and it can be seen that this enlarged protrusion is a spine (C, arrow in Fig. 4).

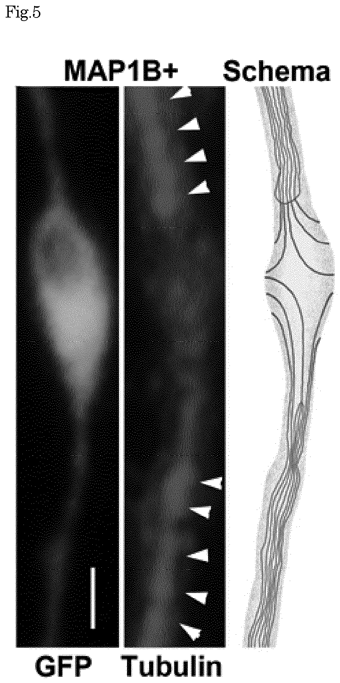

In the above-mentioned cultured hippocampal neurons, the spines enlarged by the microtubule-associated protein 1B gene bound to the lentiviral vector were simultaneously stained with microtubules. It can be seen that a part of the dendrites is enlarged on the left (Fig. 5). Red is microtubule staining, but microtubules are deeply stained on the axis of the dendrites that are not enlarged as spines (red, arrowhead). In the part that was enlarged as a spine, the microtubules of the axis were thinned. From this, it is inferred that in the part that is expanding as a spine, the microtubule of the axis extends laterally and the pressure of the microtubule to this side causes the expansion of the spine (the right is a schema).

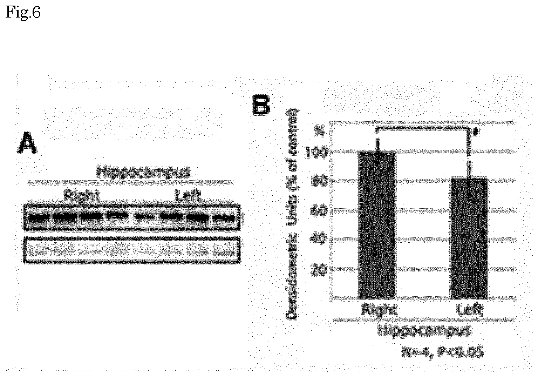

In Alzheimer's disease, the number of spines has been found to be reduced, which is determined by form. It was found that the same thing can be detected by the biochemical method of measuring the target protein, which is electrophoresis → western blotting. The lentiviral vector-microtubule-associated protein 1B gene was injected into the hippocampus only on the right side of the model mouse used this time, and one week later, the left and right hippocampi were taken out and electrophoresed (SDS-PAGE). After being transferred to a PVDF membrane or the like by a conventional method, when reacted with an antibody that recognizes a spine, that is, a protein at the posterior synapse, Right becomes thicker as shown on the left A, showing that the reduced protein in the spines of the group was restored in the group into which microtubule-associated protein 1B gene is injected (Fig. 6). In Fig. 6B, A is quantified by a densitometer, and it can be seen that right is higher in N = 4, at 5% significant.

Types of gene therapy vectors, in viral vectors (gene transfer methods that utilize the mechanism by which the virus infects cells), include, but are not limited to, retro, adeno, lenti, adeno-associated virus (AAV), and Sendai virus etc.as non-proliferative viral vectors; proliferative viral vectors include oncolytic viruses, etc.; non-viral vectors include, but are not limited to, plasmids (Naked DNA, complexes with liposomes or polymers) and bacterial vectors (genetically modified bacteria).

The viral vector used in the present invention may be retro, adeno, wrench, adeno-associated virus (AAV), Sendai virus or the like, and the non-viral vector may be a plasmid, a bacterial vector or the like. Further, instead of the conventional so-called vector, a blood-brain barrier (BBB) crossing nanomachine may be used. In short, the vector is not particularly limited as long as the gene can be expressed in brain nerve cells as long as this purpose can be achieved.

According to the present invention, it has the effect of dramatically improving dementia of Alzheimer's disease as a causal treatment rather than a symptomatic treatment. When using a vector, it must be injected into the brain by a small operation, but when using a blood-brain barrier crossing nanomachine, it can be made to act in the brain only by injection into a blood vessel.

### Examples

### (Example 1)

A method in which a mouse microtubule-associated protein 1B gene bound with a lentiviral vector is stereotactically injected into the right hippocampus by puncturing in the skull of an Alzheimer's disease model mouse to improve dementia in Alzheimer's disease.

The mouse microtubule-associated protein 1B gene was cloned as follows. Total RNA was prepared from the brain of BL6 mice using the RNeasy Mini Kit (Quiagen). The total RNA was then reverse transcribed by SuperScript III (Invitrogen) using poly T primers. A cDNA library was constructed by a standard method and the Map1b gene was cloned by screening using the Map1b gene fragment as a probe, whose nucleotide sequence was confirmed. The mouse microtubule-associated protein 1B gene (the protein coding region of the Map1b gene) was bound to a lentiviral vector using the Lenti-X TM 293T Cell Line and the Lenti-X HTX Packaging System (Clontech). 1 microliter of this chimeric gene solution was injected into model mouse B6SJLTg (APPSwFlLon, PSEN1 * M146L * L286V) 6799Vas / Mmjax (Purchased from The Jackson Laboratory, https://www.mmrrc.org/about/mmrrcContacts.php), 034840-JAX-HEMI-F - Hemizygous female, (Oakley, et al., J Neurosci. 2006 26:10129-40) with a local injection device in the brain at 2 mm to the right, 2 mm behind, from a coronal sagittal suture crossing and 1 mm from the brain surface by a cranial fixation stereotactic intracerebral local injection device.

A hole is opened on the skull of a double transgenic mouse with amyloid precursor protein gene and presenilin gene (total 5 mutations) using mouse skull fixation, microinjection system (Narikige, Tokyo, Japan), it was stereotactically injected into the right hippocampus (2 mm to the right, 2 mm behind from of bregma, and 1 mm from the brain surface). The change in the degree of dementia in Alzheimer's disease model mice measured by the Y maze test at this time is shown. By injecting the mouse microtubule-associated protein 1B gene with a viral vector into the hippocampus, microtubule-associated protein 1B was overexpressed in hippocampal neurons. In two days, it was confirmed that the degree of dementia in the injection group was clearly improved (Fig. 3).

### (Example 2)

### Confirmation by Western blotting

In Alzheimer's disease, the number of spines has been found to be reduced, which is determined by form. It was found that the same thing can be detected by the biochemical method of measuring the target protein, which is electrophoresis → western blotting. The transformation model mouse B6SJLTg (APPSwFlLon, PSEN1 * M146L * L286V) 6799Vas / Mmjax used this time was injected with the lentiviral vector-microtubule-associated protein 1B gene into the right hippocampus, and one week later, the left and right hippocampi were taken out and electrophoresed. (SDS-PAGE).

That is, the hippocampus was homogenized in 1 ml Eppendorf in a double volume of 4x laemli sample buffer (Laemmli sample buffer, Bio-Rad, Tokyo) containing a protease inhibitor, 10-20 microliter of which was electrophoresed in a 12% miniprothean TGX gel (Bio-Rad). After transfer to polyvinylidene difluoride membrane (PVDF membrane), it was reacted using primary antibody (HOMER1 rabbit polyclonal antibody 1: 500, Proteintec Group Inc., Rosemont, IL, USA) that recognizes spines, that is, proteins located at the postsynapse, and anti-tublin (1: 10000, DM1A, Sigma) as an internal control., St. Lois, MO). The PVDF membrane was then incubated with a secondary antibody ligated with horseradish peroxidase (Amersham), and the signal was detected by Image Quant 400 (GE Healthcare, Buckinghamshire, UK). Quantitative band patterns were obtained by ImageJ (NIH). The results showed that Right became thicker, as shown in the left A, and that the reduced protein in the spines of the microtubule-associated protein 1B gene-injected group was recovered (Fig. 6).

Since the method for producing a mouse microtubule-associated protein 1B gene bound to a lentiviral vector has already been described, a method for producing a mouse microtubule-associated protein 1B gene bound to a blood-brain barrier (BBB) crossing-brain barrier nanomachine is described.

Micellized nanoparticles (polymer micelles) are composed of block copolymer (copolymer) made by binding a hydrophilic polymer composed of polyethylene glycol (PEG), which has a property of being easily soluble in water, and a hydrophobic polymer composed of a polyamino acid having a property of being hard to dissolve in water at the molecular level. When the block copolymer is diffused in water, it forms micelles, which are spherical aggregates of 20 to 100 nanometer size with a clear two-layer structure of hydrophilic polymer on the outside and hydrophobic polymer on the inside. Drugs and bioactive substances can be encapsulated in the hydrophobic inner core of the micelles. It is possible to deal with various compounds by chemically changing the type and structure of amino acids. Stability in blood can be ensured by covering the surface with PEG (polyethylene glycol). In the brain, there is a special tissue called the blood-brain barrier that separates the blood from the brain tissue, and it has the function of controlling the material transport between the circulating blood and the cranial nerve system, mainly by the cerebral vascular endothelial cells ; although it selectively takes in nutrients essential for its activities, it is a tissue that can be said to be a biological barrier that significantly limits the delivery of drugs to the brain. While it selectively takes in glucose, which is essential for brain activity it significantly limits the delivery of drugs. Against this background, Kazunori Kataoka et al. have developed a cerebral blood-brain barrier crossing nanomachine that crosses the blood-brain barrier with extremely high efficiency by changing in blood glucose levels and accumulates in nerve cells in the brain (Nature Communications 8, Article number 1001, 2017).

By attaching a glucose molecule to the end of the hydrophobic block, the drug can be efficiently delivered into the brain by a simple method of injecting nanomachines on an empty stomach and then eating. By binding microtubule-associated protein 1B gene to such a nanomachine, by injecting it intravenously, and then by passing it through the blood-brain barrier by raising the blood glucose level, microtubule-associated protein 1B is expressed in brain nerve cells and dementia can be prevented or treated.

### Industrial applicability

Since those in which the microtubule-associated protein 1B gene is bound to a blood-brain barrier (BBB, blood-brain barrier) cross-type nanomachine and those in which a vector is bound to the microtubule-associated protein 1B gene of the present invention can be used for treating human Alzheimer's disease and can be used in the medical industry.

### Description of the reference numerals

1. Vector
2. Human
3. BBB passage type nanomachine
4. promoter
5. Microtubule-associated protein 1B gene (ORF. Here, the microtubule-related protein 1B gene has the same meaning)

## Claims

1. A therapeutic agent for dementia comprising any one of
i) DNA encoding microtubule-associated protein 1B (MBP 1B);
ii) A gene encoding a protein having 70% or more homology with the DNA encoding MBP 1B and having the equivalent function;
iii) DNA that hybridizes with the DNA encoding MBP1B under stringent conditions and encodes a protein having the equivalent function as the MBP1B protein.;
iv) DNA encoding a protein that has 60% or more identity with the amino acid sequence of the MBP1B protein and has the equivalent function as the MBP1B protein.

2. The dementia therapeutic agent according to claim 1, which is functionally linked to a vector.

3. The therapeutic agent for dementia according to claim 1, which is **characterized by** being bound to a blood-brain barrier (BBB) crossing nanomachine.
